# EUROPEAN PATENT APPLICATION

(11) **EP 4 184 528 A1**
(43) Date of publication of application: **24.05.2023**
(21) Application number: 21842595.7
(22) Date of filing: 13.07.2021
(51) Int. Cl.: G21G 4/08, C22B 60/02, C22B 3/24, A61K 51/00, B01J 20/22, B01J 20/34

(54) **METHOD FOR PRODUCING 225AC SOLUTION**

(30) Priority: 17.07.2020 JP 2020123131
(71) Applicant: Nihon Medi-Physics Co., Ltd, Koto-ku Tokyo, 136-0075 (JP)
(72) Inventor: HONDA, Yoshio, Tokyo 136-0075 (JP); NATSUSAKO, Masashi, Tokyo 136-0075 (JP); ASO, Shunichi, Tokyo 136-0075 (JP)
(74) Representative: TBK
(86) International application number: PCT/JP2021/026222
(87) International publication number: WO 2022/014555

(57) **Abstract**

A method for producing ²²⁵Ac solution includes steps (I) to (III): a step (I) of passing a solution (1) containing ²²⁶Ra and ²²⁵Ac through a solid-phase extraction agent (a) that contains a compound represented by formula (A) so as to cause the solid-phase extraction agent (a) to retain ²²⁵Ac; a step (II) of passing a liquid containing an eluate (2), which is obtained by eluting the retained ²²⁵Ac from the solid-phase extraction agent (a), through a solid-phase extraction agent (b) that contains a compound represented by formula (B) so as to cause the solid-phase extraction agent (b) to retain ²²⁵Ac; and a step (III) of eluting the retained ²²⁵Ac from the solid-phase extraction agent (b) to obtain an ²²⁵Ac solution. 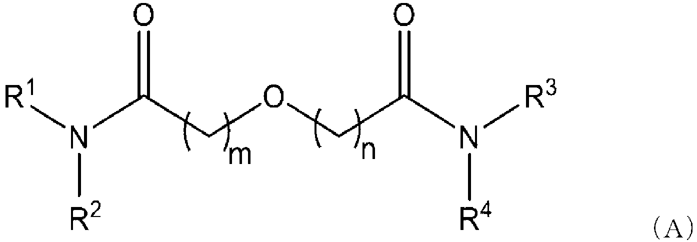 [m and n each independently represent 0 or 1, and R¹ to R⁴ each independently represent a linear or branched C8-C12 alkyl group.] 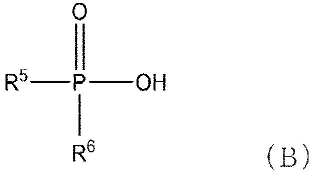 [R⁵ and R⁶ each independently represent a C8 alkyl group or a C8 alkoxy group.]

## Description

### Technical Field

An aspect of the present invention relates to a method for producing ²²⁵Ac solution.

### Background Art

In the field of nuclear medicine, radionuclide therapy has been performed in which a drug containing a radioisotope (RI) is selectively taken into a lesion such as a tumor for treatment. Among radiations, alpha-ray has a characteristic that the effect of unnecessary exposure on the surrounding normal cells is small because the range is short. ²²⁵Ac being one of the alpha-ray-emitting nuclides is a radionuclide with a half-life of 10 days, and has expected as a therapeutic nuclide in a cancer treatment and the like in recent years.

²²⁵Ac is, produced by a nuclear reaction of (p, 2n), for example, by irradiating a ²²⁶Ra target with a proton using an accelerator. Patent Literature 1 discloses a method for separation and that purification of an ²²⁵Ac component from a solution containing ²²⁶Ra ions and ²²⁵Ac ions, which is obtained by dissolving the ²²⁶Ra target after irradiation.

### Citation List

### Patent Literature

Patent Literature 1: JPA 2009-527731

### Summary of Invention

However, it has been found that conventional methods such as that described in Patent Literature 1 have at least one of the following problems.

It has been found that, according to conventional methods, a substantial amount of ²²⁶Ra remains in the ²²⁵Ac solution obtained, and thus the ²²⁵Ac purity of the ²²⁵Ac solution obtained remains to be improved.

The amount of ²²⁵Ac generated from a ²²⁶Ra target is minute, and most ²²⁶Ra remains unreacted; however, since ²²⁶Ra is a precious nuclide and is not easy to discard, the ²²⁶Ra solution after separation of ²²⁵Ac is frequently recovered (hereinafter, this recovered solution may be referred to as the "Ra recovered solution") and reused. It has been found that, according to the conventional methods, a large amount of solvent must be used to separate ²²⁶Ra and ²²⁵Ac, and thus the amount of the solvent used and the dilution of the Ra recovered solution remain to be improved.

One aspect of the present invention provides a method for producing ²²⁵Ac solution that can produce an ²²⁵Ac solution having a high ²²⁵Ac purity even when the amount of the solvent used in separating ²²⁵Ac ions from a solution containing ²²⁶Ra ions and ²²⁵Ac ions (while obtaining a Ra recovered solution having a high ²²⁶Ra concentration) is small.

The present inventors have conducted extensive studies to solve the problems described above, and found that the problems can be solved by employing a particular production method, and thus the present invention has been made.

An aspect of the present invention provides a method for producing ²²⁵Ac solution that includes steps (I) to (III) below:
step (I): a step of passing a Ra-Ac solution (1) containing ²²⁶Ra ions and ²²⁵Ac ions through a solid-phase extraction agent (a) that contains a compound represented by formula (A) below so as to cause the solid-phase extraction agent (a) to retain the ²²⁵Ac ions;
step (II): a step of eluting the ²²⁵Ac ions retained on the solid-phase extraction agent (a) from the solid-phase extraction agent (a) by using an acid-containing eluent (a), and passing a liquid containing an obtained eluate (2) through a solid-phase extraction agent (b) that contains a compound represented by formula (B) below so as to cause the solid-phase extraction agent (b) to retain the ²²⁵Ac ions; and
step (III): a step of eluting the ²²⁵Ac ions retained on the solid-phase extraction agent (b) from the solid-phase extraction agent (b) by using an acid-containing eluent (b) so as to obtain an ²²⁵Ac solution having a higher ²²⁵Ac purity than the Ra-Ac solution (1).

Another aspect of the present invention provides a method for producing ²²⁵Ac solution that includes steps (Ia) and (IIa) below:
step (Ia): a step of passing a Ra-Ac solution (1) containing ²²⁶Ra ions and ²²⁵Ac ions through a solid-phase extraction agent (a) that contains a compound represented by formula (A1) below so as to cause the solid-phase extraction agent (a) to retain the ²²⁵Ac ions; and
step (IIa): a step of eluting the ²²⁵Ac ions retained on the solid-phase extraction agent (a) from the solid-phase extraction agent (a) by using an acid-containing eluent (a).

A yet another aspect of the present invention provides a production method for producing ²²⁵Ac that includes
an irradiating step of irradiating a ²²⁶Ra target with at least one kind selected from charged particles, photons, and neutrons to generate ²²⁵Ac by nuclear reaction;
a step of dissolving, in an acidic solution, the ²²⁶Ra target irradiated in the irradiating step to obtain a Ra-Ac solution (1) that contains ²²⁶Ra ions and ²²⁵Ac ions; and
a step of obtaining an ²²⁵Ac solution by using the method for producing ²²⁵Ac solution described above.

According to an aspect of the present invention, an ²²⁵Ac solution having a high ²²⁵Ac purity can be produced from a solution that contains ²²⁶Ra ions and ²²⁵Ac ions.

According to another aspect of the present invention, an ²²⁵Ac solution having a high ²²⁵Ac purity can be produced from a solution containing ²²⁶Ra ions and ²²⁵Ac ions by using a small amount of a solvent in separating the ²²⁵Ac ions (while obtaining a Ra recovered solution having a high ²²⁶Ra concentration).

By using an ²²⁵Ac solution having a high ²²⁵Ac purity, ²²⁵Ac can be efficiently used for the desired usage, and, for example, radioactive medicines having a higher ²²⁵Ac purity and containing less contaminants such as ²²⁶Ra and daughter nuclides thereof can be produced.

### Description of Embodiments

### [Method for producing ²²⁵Ac solution]

An embodiment of the method for producing ²²⁵Ac solution according to one aspect of the present invention includes steps (I) to (III) below:
step (I): passing a Ra-Ac solution (1) containing ²²⁶Ra ions and ²²⁵Ac ions through a solid-phase extraction agent (a) that contains a compound represented by formula (A) below so as to cause the solid-phase extraction agent (a) to retain the ²²⁵Ac ions;
step (II): eluting the ²²⁵Ac ions retained on the solid-phase extraction agent (a) from the solid-phase extraction agent (a) by using an acid-containing eluent (a) and passing the obtained eluate (2) through a solid-phase extraction agent (b) that contains a compound represented by formula (B) below so as to cause the solid-phase extraction agent (b) to retain the ²²⁵Ac ions; and
step (III): eluting the ²²⁵Ac ions retained on the solid-phase extraction agent (b) from the solid-phase extraction agent (b) by using an acid-containing eluent (b) so as to obtain an ²²⁵Ac solution having a higher ²²⁵Ac purity than the aforementioned Ra-Ac solution (1).

### < Step (I) >

In the step (I), a Ra-Ac solution (1) containing ²²⁶Ra ions and ²²⁵Ac ions is passed through a solid-phase extraction agent (a) that contains a compound represented by formula (A) below so as to cause the solid-phase extraction agent (a) to retain the ²²⁵Ac ions.

By performing the step (I), the ²²⁵Ac ions can be retained on the solid-phase extraction agent (a), and the ²²⁶Ra ions not retained on the solid-phase extraction agent (a) can pass through. The flow-through solution obtained in the step (I) contains most of ²²⁶Ra ions contained in the Ra-Ac solution (1), and thus is preferably recovered and reused. Thus, typically, the flow-through solution of this step (I) constitutes a Ra recovered solution. Here, the Ra recovered solution refers to the flow-through solution in the step (I) in the following first purification step, and does not refer to the flow-through solution of a step that corresponds to the step (I) in a second purification step described below or in an optional third or onward purification step described below.

The Ra recovered solution is, for example, subjected to steps such as a purification step as necessary, and then used as, for example, an electrodeposition solution for producing a ²²⁶Ra target.

The amounts of the ²²⁶Ra ions contained in the flow-through solution passing through the solid-phase extraction agent (b) in the step (II) below and in a wash solution obtained in the washing step described below are usually extremely small compared to the amount of the ²²⁶Ra ions in the aforementioned Ra recovered solution; thus, such a flow-through solution and a wash solution are usually not reused and are usually discarded since the disadvantage of reducing the ²²⁶Ra ion concentration overweighs the advantage of the reuse. Thus, in the present description, such a flow-through solution and such a wash solution are not referred to as the Ra recovered solutions.

The solid-phase extraction agent (a) can selectively retain the ²²⁵Ac ions by passing a high concentrated acid (for example, 0.3 M or higher for nitric acid), and can thus cause ²²⁶Ra ions to pass through. As described above, the concentration of the acid used to separate ²²⁶Ra ions and ²²⁵Ac ions (causing ²²⁵Ac ions to be retained on the solid-phase extraction agent (a) and allowing ²²⁶Ra ions to pass through) by using the solid-phase extraction agent (a) is high; thus, the amount of the solvent necessary for separating the ²²⁶Ra ions and ²²⁵Ac ions is decreased. Accordingly, when the solid-phase extraction agent (a) is used in this step (I), the ²²⁶Ra ions and the ²²⁵Ac ions can be satisfactorily separated even when the amount of the solvent used to separate ²²⁵Ac ions from the solution containing ²²⁶Ra ions and ²²⁵Ac ions is small.

### << Ra-Ac solution (1) >>

The Ra-Ac solution (1) is not particularly limited as long as ²²⁶Ra ions and ²²⁵Ac ions are contained, but is preferably a solution that contains ²²⁶Ra ions, ²²⁵Ac ions, and an acid.

A preferable example of an embodiment of the Ra-Ac solution (1) is a solution obtained by irradiating a ²²⁶Ra target with at least one kind selected from charged particles, photons, and neutrons and preferably with protons, and then dissolving the ²²⁶Ra target after the irradiation. Irradiating the ²²⁶Ra target with particles generates ²²⁵Ac through, for example, decay in some cases, and thus the solution obtained by dissolving the ²²⁶Ra target contains ²²⁶Ra ions and ²²⁵Ac ions. Here, in dissolving the ²²⁶Ra target, an acid may be used.

Examples of the acid include inorganic acids such as nitric acid, hydrochloric acid, phosphoric acid, sulfuric acid, boric acid, and hydrofluoric acid. Among these, from the viewpoint such as the capability to sufficiently dissolve ²²⁶Ra ions and ²²⁵Ac ions and efficiently carry out separation purification using a solid-phase extraction agents (a) and (b), nitric acid and hydrochloric acid are preferable, and nitric acid is particularly preferable.

One acid or two or more acids may be used in the Ra-Ac solution (1).

When nitric acid is used as the aforementioned acid, from the viewpoint such as the capability to more efficiently separate ²²⁶Ra and ²²⁵Ac by passing the solution through the solid-phase extraction agent (a) (perform separation with a less amount of ²²⁵Ac passing through and a less amount of ²²⁶Ra retained), the acid concentration of the Ra-Ac solution (1) is preferably 0.3 M or higher and more preferably 0.5 M or higher, and is preferably 4.0 M or lower.

When hydrochloric acid is used as the aforementioned acid, the acid concentration of the Ra-Ac solution (1) is preferably 1 M or higher and preferably 8 M or lower.

In order to dissolve the ²²⁶Ra target, the amount of the solvent is preferably at least 10 times and more preferably at least 20 times the molar amount of ²²⁶Ra, and is preferably at most 50 times and more preferably at most 40 times the molar amount of ²²⁶Ra; for example, when 25 mg of ²²⁶Ra is to be dissolved, the amount of the acid used having the aforementioned concentration range is preferably 1 mL or more, more preferably 2 mL or more, and yet more preferably 3 mL or more, and is preferably 20 mL or less, more preferably 15 mL or less, and yet more preferably 10 mL or less.

The flow rate of the Ra-Ac solution (1) passing through the solid-phase extraction agent (a) is preferably 0.01 mL/min or more, more preferably 0.1 mL/min or more, and yet more preferably 0.5 mL/min or more, and is preferably 5 mL/min or less, more preferably 3 mL/min or less, and yet more preferably 2 mL/min or less from the viewpoint such as the capability to more efficiently separate ²²⁶Ra and ²²⁵Ac.

### << Solid-phase extraction agent (a) >>

The solid-phase extraction agent (a) is not particularly limited as long as the solid-phase extraction agent (a) contains a compound represented by formula (A) below, and may further contain known components contained in solid-phase extraction agents.

The solid-phase extraction agent (a) may be solely composed of a compound represented by formula (A), or may contain a compound represented by formula (A) below and other components (for example, known additives and inactive substrates) (including a solid-phase extraction agent that contains an inactive substrate and a compound represented by formula (A) introduced into the inactive substrate).

The solid-phase extraction agent (a) may contain one or more compounds represented by formula (A) below.

The solid-phase extraction agent (a) is preferably an inactive substrate that contains a compound represented by formula (A) below, and more preferably a porous silica or an organic polymer that contains a compound represented by formula (A) below. The pore size of the porous silica is not particularly limited but is preferably about 50 to 150 um in diameter.

The solid-phase extraction agent (a) can selectively retain the ²²⁵Ac ions by passing a high concentrated acid, and the retained ²²⁵Ac ions can be eluted by passing a low concentration acid through the solid-phase extraction agent (a) .

Such a solid-phase extraction agent (a) is not particularly limited, and a commercially available product, such as "DGA Resin" and "DGA Branched Resin" produced by Eichrom Technologies Inc., may be used.

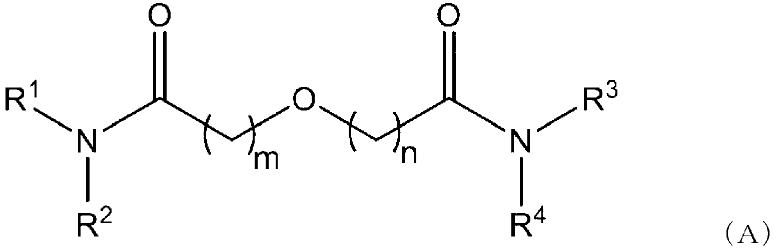

In formula (A), m and n each independently represent 0 or 1, and preferably 1.

In formula (A), R¹ to R⁴ each independently represent a linear or branched alkyl group having 8 or more and 12 or less carbon atoms. R¹ to R⁴ preferably each independently represent an octyl group or a 2-ethylhexyl group.

### < Other steps >

After the step (I) and before the step (II) described below, a step of washing the solid-phase extraction agent (a), which is retaining the ²²⁵Ac ions, with an acid-containing wash solution (solid-phase extraction agent (a) washing step) may be performed for purposes such as washing away the ²²⁶Ra ions that may remain in the solid-phase extraction agent (a).

Examples of the acid used in the solid-phase extraction agent (a) washing step are the same as those for the acid used in the Ra-Ac solution (1) described above, and preferable acids are also the same. One acid or two or more acids may be used.

The concentration of this acid is not particularly limited as long as the retained ²²⁵Ac ions do not elute, but is preferably the same or about the same as the concentration of the Ra-Ac solution (1) described above.

### < Step (II) >

In step (II), the ²²⁵Ac ions retained on the solid-phase extraction agent (a) are eluted from the solid-phase extraction agent (a) by using an acid-containing eluent (a), and a liquid containing the obtained eluate (2) is passed through a solid-phase extraction agent (b) that contains a compound represented by formula (B) below so as to cause the solid-phase extraction agent (b) to retain ²²⁵Ac ions.

By performing the step (II), the ²²⁵Ac ions contained in the eluate (2) can be retained on the solid-phase extraction agent (b), and the ²²⁶Ra ions that can be contained in the eluate (2) can be allowed to pass through.

In eluting ²²⁵Ac ions from the solid-phase extraction agent (a), an acid-containing eluent (a) is used. Examples of the acid are the same as those for the acid used in the Ra-Ac solution (1) described above, and preferable acids are also the same. One acid or two or more acids may be used.

The acid concentration of the eluent (a) is not particularly limited as long as the retained ²²⁵Ac ions can be sufficiently eluted from the solid-phase extraction agent (a); however, when the same acid as that used with the Ra-Ac solution (1) described above is used as the acid used in the eluent (a), the difference in concentration is preferably large. When nitric acid is used as the acid, the acid concentration of the eluent (a) is preferably 0.2 M or lower, more preferably 0.1 M or lower, and yet more preferably 0.01 M or lower, and the lower limit of the concentration may be any as long as nitric acid is contained, in other words, as long as the nitric acid concentration is greater than 0 M.

When hydrochloric acid is used as the aforementioned acid, the acid concentration of the eluent (a) is preferably greater than 0 M and 0.2 M or lower.

In addition, since there is a possibility that the acid used in the Ra-Ac solution (1) would remain in the solid-phase extraction agent (a), in order to ensure, for example, elution of ²²⁵Ac ions from the solid-phase extraction agent (a) in such a case also, the concentration of the acid used in the Ra-Ac solution (1) is preferably different from the acid concentration of the eluent (a), and is preferably 15 or more provided that the acid concentration of the eluent (a) is 1.

The flow rate of the eluent (a) is preferably 0.1 mL/min or more and more preferably 0.5 mL/min or more, and preferably 20 mL/min or less and more preferably 10 mL/min or less from the viewpoint such as the capability to sufficiently elute the retained ²²⁵Ac ions from the solid-phase extraction agent (a).

The obtained eluate (2) may be directly passed through the solid-phase extraction agent (b) or adjusted for the acid concentration, the flow rate and the like, and then passed through the solid-phase extraction agent (b).

By passing the eluate (2) having an acid concentration in the aforementioned range through the solid-phase extraction agent (b), ²²⁵Ac ions can be retained on the solid-phase extraction agent (b), and thus the acid concentration of the obtained eluate (2) need not be adjusted.

The flow rate in which the liquid containing the eluate (2) passes through the solid-phase extraction agent (b) is preferably 1 mL/min or more and more preferably 1.5 mL/min or more, and preferably 30 mL/min or less and more preferably 20 mL/min or less from the viewpoint such as the capability to sufficiently retain ²²⁵Ac ions on the solid-phase extraction agent (b).

### << Solid-phase extraction agent (b) >>

The solid-phase extraction agent (b) is not particularly limited as long as the solid-phase extraction agent (b) contains a compound represented by formula (B) below, and may further contain known components contained in solid-phase extraction agents.

The solid-phase extraction agent (b) may be solely composed of a compound represented by formula (B), or may contain a compound represented by formula (B) below and other components (for example, known additives and inactive substrates) (including a solid-phase extraction agent that contains an inactive substrate and a compound represented by formula (B) introduced into the inactive substrate).

The solid-phase extraction agent (b) may contain one or more compounds represented by formula (B) below.

The solid-phase extraction agent (b) is preferably an inactive substrate that contains a compound represented by formula (B) below, and more preferably a porous silica or an organic polymer that contains a compound represented by formula (B) below. The pore size of the porous silica is not particularly limited but is preferably about 50 to 150 um in diameter.

The solid-phase extraction agent (b) can selectively retain the ²²⁵Ac ions by passing a low concentration acid, and the retained ²²⁵Ac ions can be eluted by passing a high concentrated acid through the solid-phase extraction agent (b) .

Such a solid-phase extraction agent (b) is not particularly limited, and a commercially available product, such as "Ln Resin", "Ln2 Resin", and "Ln3 Resin" produced by Eichrom Technologies Inc., may be used.

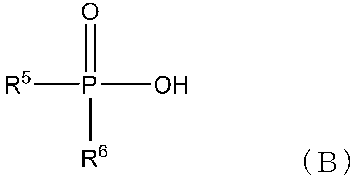

In formula (B), R⁵ and R⁶ each independently represent - R' or -OR' (R' represents an alkyl group having 8 carbon atoms). The alkyl group having 8 carbon atoms represented by R' may be linear or branched, and preferable examples thereof include an octyl group, a 2-ethylhexyl group, and 2-methyl-4,4-dimethylpentyl group.

Preferable examples of the compound represented by formula (B) are compounds represented by formulae (B-1) to (B-3) below.

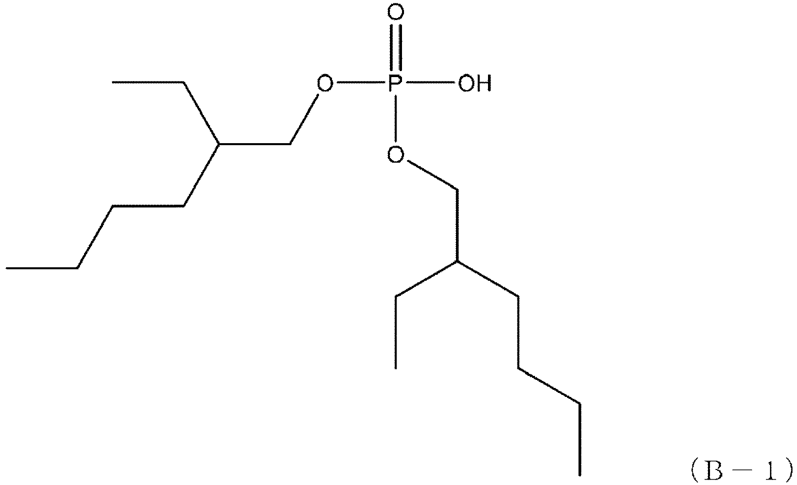

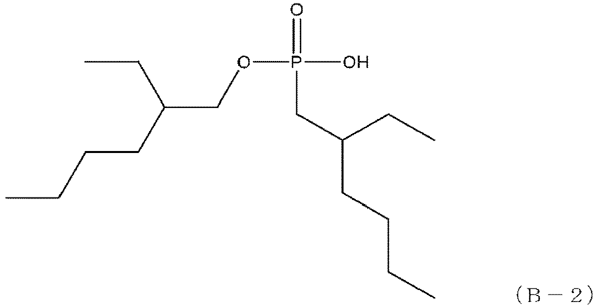

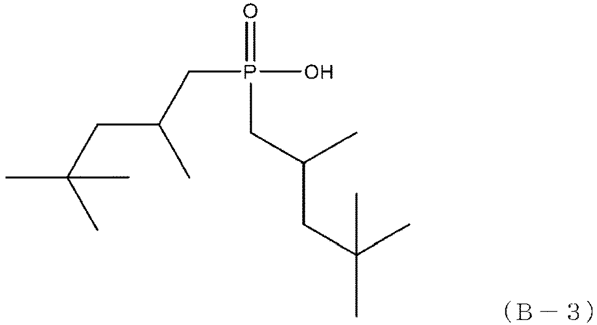

### < Other steps >

After the step (II) and before the step (III) described below, for example, a step of washing the solid-phase extraction agent (b), which is retaining the ²²⁵Ac ions, with an acid that has an acid concentration higher than the liquid containing the eluate (2) but lower than the acid concentration of the eluent (b) below (solid-phase extraction agent (b) washing step) may be performed for the purposes such as washing away ²²⁶Ra ions that may remain in the solid-phase extraction agent (b).

This solid-phase extraction agent (b) washing step is preferably performed separately from the step (II) described above; however, if ²²⁶Ra ions had been sufficiently removed in the aforementioned solid-phase extraction agent (a) washing step, the concentration of the eluent (a) and the concentration of the acid in the eluate (2) may be adjusted so that the step of passing the liquid containing the eluate (2) through the solid-phase extraction agent (b) would simultaneously serve as the solid-phase extraction agent (b) washing step.

From the viewpoint such as the capability to obtain an ²²⁵Ac solution having a high ²²⁵Ac purity, the solid-phase extraction agent (b) washing step is preferably performed after the step (II).

Examples of the acid used in the solid-phase extraction agent (b) washing step are the same as those for the acid used in the Ra-Ac solution (1) described above, and preferable acids are also the same. One acid or two or more acids may be used.

The acid concentration is preferably such that the ²²⁶Ra ions are eluted but the retained ²²⁵Ac ions are not, and is preferably higher than the acid concentration of the liquid containing the eluate (2) but lower than the acid concentration of the eluent (b) below. When nitric acid is used as the acid, the concentration is preferably 0.01 M or higher and preferably 0.2 M or lower.

The flow rate of the liquid passing through in the solid-phase extraction agent (b) washing step is preferably 0.5 mL/min or more and more preferably 1 mL/min or more and preferably 30 mL/min or less and more preferably 20 mL/min or less from the viewpoint such as the capability to sufficiently elute ²²⁶Ra ions that may remain on the solid-phase extraction agent (b).

### < Step (III) >

In the step (III), the ²²⁵Ac ions retained on the solid-phase extraction agent (b) are eluted from the solid-phase extraction agent (b) by using an acid-containing eluent (b) so as to obtain an ²²⁵Ac solution having a higher ²²⁵Ac purity than the aforementioned Ra-Ac solution (1).

In eluting ²²⁵Ac ions from the solid-phase extraction agent (b), an acid-containing eluent (b) is used. Examples of the acid are the same as those for the acid used in the Ra-Ac solution (1) described above, and preferable acids are also the same. One acid or two or more acids may be used.

When nitric acid is used as the acid, the acid concentration of the eluent (b) is preferably 0.2 M or higher, more preferably 0.3 M or higher, and yet more preferably 0.5 M or higher, and is preferably 4 M or lower, more preferably 2 M or lower, and yet more preferably 1 M or lower from the viewpoint such as the capability to sufficiently elute the retained ²²⁵Ac ions from the solid-phase extraction agent (b).

When hydrochloric acid is used as the aforementioned acid, the acid concentration of the eluent (b) is preferably 0.3 M or higher and preferably 8 M or lower.

The flow rate of the eluent (b) is preferably 0.5 mL/min or more, more preferably 1 mL/min or more, and yet more preferably 2 mL/min or more, and preferably 30 mL/min or less, more preferably 25 mL/min or less, and yet more preferably 20 mL/min or less from the viewpoint such as the capability to sufficiently elute the retained ²²⁵Ac ions from the solid-phase extraction agent (b).

An ²²⁵Ac solution having a high ²²⁵Ac purity can be produced through the steps described above. The obtained ²²⁵Ac solution may be re-purified if necessary. An ²²⁵Ac solution having a yet higher ²²⁵Ac purity can be produced through the re-purification.

When re-purification is to be performed, the concentration of the acid used to elute ²²⁵Ac ions from the solid-phase extraction agent (b) may be the same as the concentration of the acid used to retain ²²⁵Ac ions on the solid-phase extraction agent (a); thus, the eluate from the solid-phase extraction agent (b) can be directly passed through the solid-phase extraction agent (a) to allow the solid-phase extraction agent (a) to retain the ²²⁵Ac ions. Thus, re-purification can be performed easily without adjusting the concentration of the acid, and an ²²⁵Ac solution having a yet higher ²²⁵Ac purity can be produced.

The re-purification preferably includes a first purification step of obtaining an ²²⁵Ac solution by performing the aforementioned steps (I) to (III) and other steps described above, and a second purification step of re-purifying the ²²⁵Ac solution obtained in the first purification step.

The second purification step may be part of the first purification step, may be the same as the first purification step, or may be a step that repeats these steps.

The second purification step preferably includes: a step of passing the ²²⁵Ac solution, which is obtained in the first purification step, through the same solid-phase extraction agent (a) used in the first purification step or a different solid-phase extraction agent (a) (a solid-phase extraction agent (a) that contains a compound represented by formula (A) above) so as to cause the solid-phase extraction agent (a) to retain ²²⁵Ac ions; and
a step of eluting the ²²⁵Ac ions retained on the solid-phase extraction agent (a) from the solid-phase extraction agent (a) by using an acid-containing eluent so as to obtain an ²²⁵Ac solution having a higher ²²⁵Ac purity than the ²²⁵Ac solution obtained in the first purification step.

The method for producing ²²⁵Ac solution that includes such a second purification step is preferable since the acid concentration in the obtained ²²⁵Ac solution is low and thus the ²²⁵Ac solution can be directly used in labeling reaction, for example.

In addition, the second purification step may further include: a step of passing the ²²⁵Ac solution, which is obtained by elution from the solid-phase extraction agent (a) as described above, through the same solid-phase extraction agent (b) used in the first purification step or a different solid-phase extraction agent (b) (a solid-phase extraction agent (b) that contains a compound represented by formula (B) above) so as to cause the solid-phase extraction agent (b) to retain ²²⁵Ac ions; and
a step of eluting the ²²⁵Ac ions retained on the solid-phase extraction agent (b) from the solid-phase extraction agent (b) by using an acid-containing eluent so as to obtain an ²²⁵Ac solution having a yet higher ²²⁵Ac purity than the ²²⁵Ac solution obtained in the first purification step.

The method for producing ²²⁵Ac solution that includes such a second purification step increases the ²²⁵Ac purity of the obtained ²²⁵Ac solution, and thus when the ²²⁵Ac solution is appropriately adjusted for the acid concentration and used in the labeling reaction, for example, a high labeling ratio and a high synthetic yield can be achieved.

The acid-containing eluent used in the second purification step preferably has a composition similar to that of the eluent used in the corresponding first purification step, and more preferably has the same composition as the eluent used in the corresponding first purification step. Furthermore, the second purification step preferably further includes a solid-phase extraction agent (a) washing step and a solid-phase extraction agent (b) washing step, and more preferably, the same steps as the washing steps performed in the first purification step are performed.

### < Step (Ia) >

According to another embodiment of the present invention, a step (Ia) may be performed instead of the aforementioned step (I). In the step (Ia), a Ra-Ac solution (1) containing ²²⁶Ra ions and ²²⁵Ac ions is passed through a solid-phase extraction agent (a) that contains a compound represented by formula (A1) below so as to cause the solid-phase extraction agent (a) to retain the ²²⁵Ac ions.

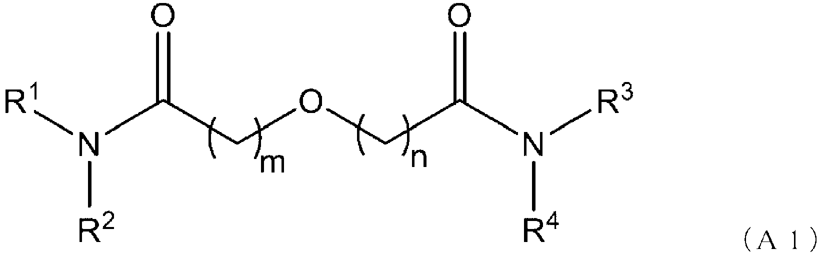

In formula (A1), m and n each independently represent 1.

In formula (A1), R¹ to R⁴ each independently represent a linear or branched alkyl group having 8 or more and 12 or less carbon atoms. R¹ to R⁴ preferably each independently represent an octyl group or a 2-ethylhexyl group.

The Ra-Ac solution (1) used in the step (Ia) may have a similar composition to the Ra-Ac solution (1) used in the step (I) described above, and the flow rate of the solution passing through the solid-phase extraction agent (a) is also similar to that of the Ra-Ac solution (1) used in the step (I) described above.

The solid-phase extraction agent (a) used in the step (Ia) is also the same as the solid-phase extraction agent (a) used in the step (I) described above except that the compound represented by formula (A1) above is used instead of the compound represented by formula (A) above.

### < Step (IIa) >

According to another embodiment of the present invention, after the step (Ia) above, a step (IIa) may be performed instead of the aforementioned steps (II) and (III). In the step (IIa), the ²²⁵Ac ions retained on the solid-phase extraction agent (a) are eluted from the solid-phase extraction agent (a) by using an acid-containing eluent (a) so as to obtain an ²²⁵Ac solution having a higher ²²⁵Ac purity than the aforementioned Ra-Ac solution (1).

The acid-containing eluent (a) used in eluting the ²²⁵Ac ions from the solid-phase extraction agent (a) in the step (IIa) can be a solution having a composition similar to that of the eluent (a) used in the step (II), and the flow rate of the solution passing through the solid-phase extraction agent (a) is also similar to that of the eluent (a) used in the step (II) described above.

### [Method for producing ²²⁵Ac]

A method for producing ²²⁵Ac according to one embodiment of the present invention includes:
a step of irradiating a ²²⁶Ra target with at least one kind selected from charged particles, photons, and neutrons to generate ²²⁵Ac by nuclear reaction;
a step of dissolving, in an acidic solution, the ²²⁶Ra target irradiated in the aforementioned irradiating step to obtain a Ra-Ac solution (1) that contains ²²⁶Ra ions and ²²⁵Ac ions; and
a step of obtaining an ²²⁵Ac solution by using the method for producing ²²⁵Ac described above.

The details of the irradiating step and the step of obtaining the Ra-Ac solution (1) are as described in the section explaining the Ra-Ac solution (1) above.

The method for producing ²²⁵Ac according to an embodiment of the present invention may further include other steps such as a step of removing the solvent from the ²²⁵Ac solution.

In one embodiment of the present invention, the ²²⁵Ac solution may be used in producing an ²²⁵Ac-containing therapeutic drug. In one embodiment of the present invention, a pharmaceutically acceptable ²²⁵Ac-containing radionuclide composition obtained by this method for producing ²²⁵Ac may be provided.

### EXAMPLES

Hereinafter, an embodiment of the present invention is further described through Examples which do not limit the present invention in any way.

Since a test that uses ²²⁶Ra cannot be easily carried out due to issues such as radiation issues, barium, which is considered to give similar results to ²²⁶Ra, was used in the test described below. Radium is an alkaline earth metal and has similar properties to barium, which is also an alkaline earth metal and has a mass number close to radium. Furthermore, radium and barium are known to have similar properties as indicated by the fact that coprecipitation action with barium sulfate has been utilized in extracting radium from a pitch blend after uranium extraction in the past.

### [Test Example 1]

### · Step (I)

²²⁵Ac ions (produced by Oak Ridge National Laboratory (ORNL)) were dissolved in a solution prepared by diluting 53.3 mL of nitric acid having a concentration of 60% with water to 1 L so as to prepare a 0.7 M aqueous nitric acid solution (radioactivity concentration of ²²⁵Ac: 0.04 to 1 MBq/run). Furthermore, 16 mL of a solution (1-1) prepared by dissolving barium chloride in the obtained aqueous solution such that the mass of Ba was 30 mg was passed through a solid-phase extraction agent (a) ("DGA Resin" produced by Eichrom Technologies Inc., DGA normal resin, 1 mL cartridge, containing a compound represented by formula (A) above) at a flow rate of 0.8 mL/min (actual measured value). By passing this solution, ²²⁵Ac ions were retained on the DGA Resin, and a flow-through solution containing Ba (this flow-through solution corresponds to the Ra recovered solution) was obtained.

Next, DGA Resin after the passage of the solution (1-1) was washed by passing 20 mL of a 0.7 M aqueous nitric acid solution therethrough at a flow rate of 0.8 mL/min (actual measured value) (the wash solution that has passed through the DGA resin is also referred to as the wash solution (x)).

The radioactivity was measured with a germanium semiconductor detector produced by EURISYS MESURES to determine the amounts of ²²⁵Ac in the obtained Ra recovered solution and the wash solution (x); however, the amounts of ²²⁵Ac were below the detection limit of the detector and ²²⁵Ac could not be detected. The measured masses of radioactive substances described below are the values measured by using a similar detector.

In addition, the result of the amount of ²²⁵Ac in Test Example 1 is an average value obtained by performing the same process three times.

### · Step (II)

An eluate (2-1) containing ²²⁵Ac ions was obtained by passing 20 mL of a 0.005 M aqueous nitric acid solution through the DGA Resin, which had been washed with the 0.7 M aqueous nitric acid solution, at a flow rate of 0.8 mL/min (actual measured value), and the obtained eluate (2-1) was passed through a solid-phase extraction agent (b) ("Ln Resin" produced by Eichrom Technologies Inc., 1 mL cartridge, containing a compound represented by formula (B) above) at a flow rate of 2.5 mL/min (actual measured value). By passing this solution, ²²⁵Ac ions were retained on the Ln Resin. The flow-through solution that has passed through Ln Resin during this process is also referred to as a flow-through solution (y).

The amount of ²²⁵Ac in the obtained flow-through solution (y) was measured and was found to be below the detection limit of the detector, and thus ²²⁵Ac could not be detected.

In addition, the amount of ²²⁵Ac in the DGA Resin after the passage of the 0.005 M aqueous nitric acid solution was measured, and the average value (n = 3) of the amount of ²²⁵Ac was 1.6% relative to 100% of the amount of ²²⁵Ac used.

### · Solid-phase extraction agent (b) washing step

Next, the Ln Resin after the passage of the eluate (2-1) was washed by passing 10 mL of a 0.05 M aqueous nitric acid solution therethrough at a flow rate of 1 mL/min (actual measured value) (the wash solution that has passed through the Ln Resin is also referred to as the wash solution (z)).

The amount of ²²⁵Ac in the obtained wash solution (z) was measured and was found to be below the detection limit of the detector, and thus ²²⁵Ac could not be detected.

### · Step (III)

10 mL of a 0.7 M aqueous nitric acid solution was passed through the Ln Resin, which had been washed with the 0.05 M aqueous nitric acid solution, at a flow rate of 2.5 mL/min (actual measure value) to obtain an ²²⁵Ac solution that contains ²²⁵Ac.

The amount of ²²⁵Ac in the obtained ²²⁵Ac solution was measured, and the average value (n = 3) of the amount of ²²⁵Ac was 98.4% relative to 100% of the amount of ²²⁵Ac used.

The amounts of ²²⁵Ac in the Ln Resin after the passage of the 0.7 M aqueous nitric acid solution and in the members such as a syringe and a tube used in the aforementioned operation were measured and were found to be below the detection limit of the detector, and thus ²²⁵Ac could not be detected.

### [Test Example 2]

A Ra recovered solution was obtained as in the step (I) described above in Test Example 1 except that, instead of 16 mL of the 0.7 M aqueous nitric acid solution containing dissolved ²²⁵Ac, 20 mL of a 4 M aqueous nitric acid solution containing dissolved ²²⁵Ac, 15 mL of a 1 M aqueous nitric acid solution containing dissolved ²²⁵Ac, or 15 mL of a 0.5 M aqueous nitric acid solution containing dissolved ²²⁵Ac (in every one of these solutions, the ²²⁵Ac concentration was 0.04 to 0.1 MBq/run) was used, and the amount of ²²⁵Ac in this Ra recovered solution was measured. The amount of ²²⁵Ac was below the detection limit of the detector when the 4 M or 1 M aqueous nitric acid solution was used, and was 0.6% relative to 100% of the amount of ²²⁵Ac used when the 0.5 M aqueous nitric acid solution was used.

### [Test Example 3]

The steps (I) and (II) were performed as described above except that, in the step (I) of Test Example 1, a 4 M aqueous nitric acid solution containing dissolved ²²⁵Ac (radioactivity concentration of ²²⁵Ac: 0.04 to 0.1 MBq/run) was used instead of the 0.7 M aqueous nitric acid solution containing dissolved ²²⁵Ac and a 4 M aqueous nitric acid solution was used as the solution for washing the DGA Resin and that, in the step (II) of Test Example 1, a liquid containing 30 mL of water and 5 mL of a 0.01 M aqueous nitric acid solution was used instead of 20 mL of the 0.005 M aqueous nitric acid solution. The amount of ²²⁵Ac in the flow-through solution (y) obtained was 0.3% relative to 100% of the amount of ²²⁵Ac used, and the amount of ²²⁵Ac in the DGA Resin after the passage of the 0.01 M aqueous nitric acid solution was 1.3% relative to 100% of the amount of ²²⁵Ac used.

### [Test Example 4]

The steps (I) and (II) were performed as described above except that, in the step (I) of Test Example 1, a 0.5 M aqueous nitric acid solution containing dissolved ²²⁵Ac (0.04 to 0.1 MBq/run) was used instead of the 0.7 M aqueous nitric acid solution containing dissolved ²²⁵Ac and that a 0.5 M aqueous nitric acid solution was used as the solution for washing the DGA Resin. The amount of ²²⁵Ac in the flow-through solution (y) obtained was below the detection limit of the detector, and the amount of ²²⁵Ac in the DGA Resin after the passage of the 0.005 M aqueous nitric acid solution was 3.4% relative to 100% of the amount of ²²⁵Ac used.

### [Test Example 5]

The same test was performed as described above except that, in the solid-phase extraction agent (b) washing step of Test Example 1 above, 10 mL of a 0.01 M aqueous nitric acid was used instead of 10 mL of the 0.05 M aqueous nitric acid solution. The amount of ²²⁵Ac in the obtained wash solution (z) was below the detection limit of the detector, and ²²⁵Ac could not be detected.

### [Test Example 6]

The same test was performed as described above except that, in the step (III) of Test Example 1 above, a 1 M or 0.5 M aqueous nitric acid solution was used instead of the 0.7 M aqueous nitric acid solution. The amount of ²²⁵Ac in the Ln Resin after the passage of the 1 M or 0.5 M aqueous nitric acid solution was below the detection limit of the detector, and ²²⁵Ac could not be detected.

### [Reference Example 1]

In order to study the behavior of ²²⁶Ra ions removed from the Ra-Ac solution (1) by the method for producing ²²⁵Ac solution according to an embodiment of the present invention, the following experiment was carried out. Here, instead of ²²⁶Ra ions, ¹³³Ba, which is considered to have a similar behavior, was used.

### · Step (I)

16 mL of a solution (1-2) obtained by dissolving ¹³³Ba in a 0.7 M aqueous nitric acid solution containing dissolved ¹³³Ba (the ¹³³Ba concentration: 0.8 MBq/run) by using barium chloride such that the mass of Ba was 15 mg was passed through a solid-phase extraction agent (a) (DGA Resin). As a result, a flow-through solution containing ¹³³Ba (this flow-through solution corresponds to the Ra recovered solution) was obtained.

Next, the DGA Resin after the passage of the solution (1-2) was washed by passing 20 mL of a 0.7 M aqueous nitric acid solution therethrough (the wash solution that has passed through the DGA resin is also referred to as the wash solution (x')).

The radioactivity was measured with a germanium semiconductor detector to determine the amounts of ¹³³Ba in the obtained ¹³³Ba-containing flow-through solution (corresponds to the Ra recovered solution) and in the wash solution (x'). The amount of ¹³³Ba was 99.8% relative to 100% of the amount of ¹³³Ba used.

### · Step (II) and solid-phase extraction agent (b) washing step

An eluate (2-2) was obtained by passing 20 mL of a 0.005 M aqueous nitric acid solution through the DGA Resin, which had been washed with a 0.7 M aqueous nitric acid solution, and the obtained eluate (2-2) was passed through a solid-phase extraction agent (b) ("Ln Resin"). The flow-through solution that has passed through the Ln Resin during this process is also referred to as a flow-through solution (y').

Next, the Ln Resin after the passage of the eluate (2-2) was washed with 10 mL of a 0.05 M aqueous nitric acid solution (the wash solution that has passed through the Ln resin is also referred to as the wash solution (z')).

The amounts of ¹³³Ba in the obtained flow-through solution (y') and in the wash solution (z') were measured, and the amount of ¹³³Ba was 0.2% relative to 100% of the amount of ¹³³Ba used.

The amount of ¹³³Ba in the DGA Resin after the passage of the 0.005 M aqueous nitric acid solution was measured, and was below the detection limit of the detector, and thus ¹³³Ba could not be detected.

### · Step (III)

10 mL of a 0.7 M aqueous nitric acid solution was passed through the Ln Resin, which had been washed with a 0.05 M aqueous nitric acid solution, to obtain a flow-through solution.

The amount of ¹³³Ba in the obtained flow-through solution was measured and was found to be below the detection limit of the detector, and thus ¹³³Ba could not be detected.

The amounts of ¹³³Ba in the Ln Resin after the passage of the 0.7 M aqueous nitric acid solution and in the members such as a syringe and a tube used in the aforementioned operation were measured and were found to be below the detection limit of the detector, and thus ¹³³Ba could not be detected.

### [Comparative Test Example 1]

5 mL of a 0.7 M aqueous nitric acid solution obtained by dissolving proton-irradiated ²²⁶Ra target and containing 80 pci of ²²⁶Ra and 0.08 pci of ²²⁵Ac was passed through a solid-phase extraction agent (a) (DGA resin). Then the DGA resin was washed with 20 mL of a 0.7 M aqueous nitric acid solution.

Subsequently, 20 mL of a 0.005 M aqueous nitric acid solution was passed through the DGA Resin, which had been washed with the 0.7 M aqueous nitric acid solution, to obtain an eluate that contained ²²⁵Ac.

The amount of ²²⁶Ra in the obtained eluate was measured and was 0.5 pci, and 0.6% of ²²⁶Ra remained relative to 100% of the amount of ²²⁶Ra used.

## Claims

1. A method for producing ²²⁵Ac solution comprising steps (I) to (III) below:
step (I): a step of passing a Ra-Ac solution (1) containing ²²⁶Ra ions and ²²⁵Ac ions through a solid-phase extraction agent (a) that contains a compound represented by formula (A) below so as to cause the solid-phase extraction agent (a) to retain the ²²⁵Ac ions;
step (II): a step of eluting the ²²⁵Ac ions retained on the solid-phase extraction agent (a) from the solid-phase extraction agent (a) by using an acid-containing eluent (a), and passing a liquid containing an obtained eluate (2) through a solid-phase extraction agent (b) that contains a compound represented by formula (B) below so as to cause the solid-phase extraction agent (b) to retain the ²²⁵Ac ions; and
step (III): a step of eluting the ²²⁵Ac ions retained on the solid-phase extraction agent (b) from the solid-phase extraction agent (b) by using an acid-containing eluent (b) so as to obtain an ²²⁵Ac solution having a higher ²²⁵Ac purity than the Ra-Ac solution (1), 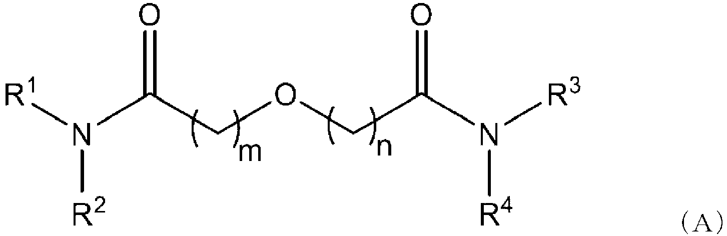 wherein, in formula (A), m and n each independently represent 0 or 1, and R¹ to R⁴ each independently represent a linear or branched alkyl group having 8 or more and 12 or less carbon atoms, 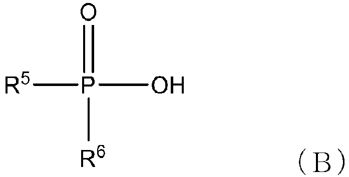 wherein, in formula (B), R⁵ and R⁶ each independently represent an alkyl group having 8 carbon atoms or an alkoxy group having 8 carbon atoms.

2. The method for producing ²²⁵Ac solution according to Claim 1, wherein the Ra-Ac solution (1) is a solution obtained by dissolving a ²²⁶Ra target that has been irradiated with at least one kind selected from charged particles, photons, and neutrons.

3. The method for producing ²²⁵Ac solution according to Claim 1 or 2, wherein the Ra-Ac solution (1) contains an acid having a concentration of 0.3 M or higher.

4. The method for producing ²²⁵Ac solution according to any one of Claims 1 to 3, wherein, after the step (I), the solid-phase extraction agent (a) retaining the ²²⁵Ac ions is washed with an acid-containing wash solution, and then the step (II) is performed.

5. The method for producing ²²⁵Ac solution according to any one of Claims 1 to 4, wherein, after the step (II), the solid-phase extraction agent (b) retaining the ²²⁵Ac ions is washed with an acid that has an acid concentration higher than that of the liquid that contains the eluate (2) but lower than that of the eluent (b), and then the step (III) is performed.

6. The method for producing ²²⁵Ac solution according to any one of Claims 1 to 5, wherein the eluent (b) has a higher acid concentration than the eluent (a).

7. The method for producing ²²⁵Ac solution according to any one of Claims 1 to 6, wherein the eluent (a) has an acid concentration of 0.2 M or lower.

8. The method for producing ²²⁵Ac solution according to any one of Claims 1 to 7, wherein the eluent (b) has an acid concentration of 0.2 M or higher.

9. The method for producing ²²⁵Ac solution according to any one of Claims 1 to 8, wherein acids contained in the eluent (a) and the eluent (b) each contain at least one inorganic acid selected from the group consisting of hydrochloric acid, nitric acid, phosphoric acid, sulfuric acid, boric acid, and hydrofluoric acid.

10. The method for producing ²²⁵Ac solution according to any one of Claims 1 to 9, wherein acids contained in the eluent (a) and the eluent (b) each contain hydrochloric acid or nitric acid.

11. The method for producing ²²⁵Ac solution according to any one of Claims 1 to 10, wherein, after the step (III), at least part of the steps (I) to (III) is further performed by using the obtained ²²⁵Ac solution so as to obtain an ²²⁵Ac solution having a yet higher ²²⁵Ac purity.

12. A method for producing ²²⁵Ac solution comprising:
a first purification step of obtaining an ²²⁵Ac solution by carrying out the method for producing ²²⁵Ac solution according to any one of Claims 1 to 10; and
a second purification step of re-purifying the ²²⁵Ac solution obtained in the first purification step,
wherein the second purification step includes:
a step of passing the ²²⁵Ac solution, which is obtained in the first purification step, through the same solid-phase extraction agent (a) used in the first purification step or a different solid-phase extraction agent (a) so as to cause the solid-phase extraction agent (a) to retain ²²⁵Ac ions, and
a step of eluting the ²²⁵Ac ions retained on the solid-phase extraction agent (a) from the solid-phase extraction agent (a) by using an acid-containing eluent so as to obtain an ²²⁵Ac solution having a yet higher ²²⁵Ac purity than the ²²⁵Ac solution obtained in the first purification step.

13. The method for producing ²²⁵Ac solution according to Claim 12, comprising:
a step of passing the ²²⁵Ac solution, which is obtained by elution from the solid-phase extraction agent (a) in the second purification step, through the same solid-phase extraction agent (b) used in the first purification step or a different solid-phase extraction agent (b) so as to cause the solid-phase extraction agent (b) to retain ²²⁵Ac ions; and
a step of eluting the ²²⁵Ac ions retained on the solid-phase extraction agent (b) from the solid-phase extraction agent (b) by using an acid-containing eluent so as to obtain an ²²⁵Ac solution having a yet higher ²²⁵Ac purity than the ²²⁵Ac solution obtained in the first purification step.

14. A method for producing ²²⁵Ac solution comprising steps (Ia) and (IIa) below:
step (Ia): a step of passing a Ra-Ac solution (1) containing ²²⁶Ra ions and ²²⁵Ac ions through a solid-phase extraction agent (a) that contains a compound represented by formula (A1) below so as to cause the solid-phase extraction agent (a) to retain the ²²⁵Ac ions; and
step (IIa): a step of eluting the ²²⁵Ac ions retained on the solid-phase extraction agent (a) from the solid-phase extraction agent (a) by using an acid-containing eluent (a), 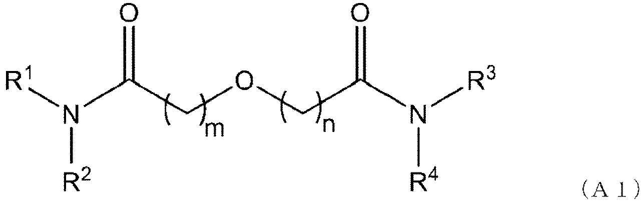 wherein, in formula (A1), m and n each independently represent 1, and R¹ to R⁴ each independently represent a linear or branched alkyl group having 8 or more and 12 or less carbon atoms.

15. An ²²⁵Ac production method comprising:
an irradiating step of irradiating a ²²⁶Ra target with at least one kind selected from charged particles, photons, and neutrons to generate ²²⁵Ac by nuclear reaction;
a step of dissolving, in an acidic solution, the ²²⁶Ra target irradiated in the irradiating step to obtain a Ra-Ac solution (1) that contains ²²⁶Ra ions and ²²⁵Ac ions; and
a step of obtaining an ²²⁵Ac solution by using the method for producing ²²⁵Ac solution according to any one of Claims 1 to 14.
